# FASCICULE DE BREVET EUROPEEN

(11) **EP 0 828 592 B1**
(45) Date de publication et mention de la délivrance du brevet: **07.08.2002**
(21) Numéro de dépôt: 96918731.9
(22) Date de dépôt: 29.05.1996
(51) Int. Cl.: B26F 3/02

(54) **MACHINE AVEC DISPOSITIF DE SEPARATION PROCEDANT PAR ARRACHAGE**
MASCHINE MIT VORRICHTUNG ZUR TRENNUNG DURCH ABREISSEN
MACHINE WITH PULLING ACTION SEPARATOR

(30) Priorité: 31.05.1995 FR 9506675
(43) Date de publication de la demande: 18.03.1998
(73) Titulaire: NATUREMBAL S.A., 67330 Bouxwiller (FR)
(72) Inventeur: BAUMULLER, Théodore, F-67590 Schweighouse-sur-Moder (FR)
(74) Mandataire: Littolff, Denis
(86) Numéro de dépôt international: FR9600805
(87) Numéro de publication internationale: WO9638272

(56) Documents cités:
- DE-C- 247 660
- FI-B- 91 839
- FR-A- 2 236 993
- US-A- 5 123 889
- US-A- 5 213 867

## Description

La présente invention concerne une machine selon le préambule de la revendication 1 et un procédé selon le préambule de la revendication 20.

Plus précisément, selon l'invention, ledit arrachage est réalisé en deux temps séquentiels : dans une première phase, le matériau de type bande est maintenu fermement immobilisé, au moins dans la zone d'arrachage et, dans une seconde phase, le dispositif opère un déchirement du matériau immobile perpendiculairement à l'axe de défilement.

Ce type de dispositif est destiné à être utilisé notamment en sortie de machines de fabrication de matériau de calage/rembourrage à partir d'un matériau de type bande de papier mono ou multicouche, dont les bordures latérales sont repliées longitudinalement sur la zone longitudinale centrale de ladite bande, l'ensemble étant ensuite entraîné, froissé, puis assemblé fermement à l'aide de compressions ponctuelles successives.

Le produit de calage/rembourrage obtenu en sortie de ce type de machine se présente sous la forme d'une bande résiliente à zone centrale d'épaisseur plus réduite que les bordures latérales, lesquelles sont en forme de coussinets longitudinaux plus lâches. Pour que le produit soit utilisable dans un processus de calage/rembourrage destiné à protéger des pièces lors de leur emballage, il faut pouvoir disposer de tronçons de longueur variable, selon le volume de l'emballage et/ou les dimensions desdites pièces. C'est pourquoi, il est nécessaire de placer des dispositifs de séparation en sortie de telles machines.

Jusqu'ici, il s'agissait généralement de dispositifs de coupe munis d'un couteau mobile, comme ceux qui apparaissent par exemple dans les documents de brevets US 4 699 609 et US 5 123 889. Dans le premier cité, l'outil de coupe est de type cisaille, avec une lame fixe et une lame mobile, actionnée par exemple par un moteur électrique. Le second brevet est présenté comme divulguant un perfectionnement dudit dispositif de coupe, car le moyen d'entraînement, combinant un moyen moteur et un système mécanique classique de type bielle-manivelle, permet un meilleur contrôle des opérations successives de coupe en transformant le mouvement rotatif du moyen moteur en un mouvement alternatif d'ordre linéaire.

Dans tous les cas, ies dispositifs de sectionnement actuels opèrent par cisaillement latéral, entraînant par conséquent l'application d'efforts latéraux par rapport à la bande de matériau à cisailler. Ces efforts s'exercent successivement sur les différentes couches du produit, lesquelles ont été auparavant solidarisées les unes aux autres par un moyen mécanique donnant une cohésion assez irrégulière auxdites couches, par exemple par embossage, gaufrage ou équivalent. Les efforts de cisaillement ont notamment pour conséquence de diminuer les effets de cette solidarisation de type à frottement pratiquée sur les reliefs des couches successives obtenues par lesdits procédés, et peuvent menacer la cohésion du produit au niveau des extrémités sectionnées.

L'invention est définie par la machine selon la revendication 1 et le procédé selon la revendication 20.

Le dispositif de sectionnement de la machine et du procédé selon la présente invention solidarise les différentes couches au niveau de chaque extrémité sectionnée, ce qui a pour effet d'améliorer très sensiblement la cohésion des produits de calage/rembourrage obtenus.

Dans un brevet français référencé FR-A-2 236 993, un dispositif opérant par déchirement est décrit, mais ce dispositif s'applique dans un contexte très différent, pour résoudre un problème distinct de celui qui fait l'objet de l'invention.

En effet, l'objectif est de sectionner par déchirement une bande monocouche en tissu fait de fibres courtes. Dans cette optique, le but du dispositif est d'éviter d'appliquer une force perpendiculaire à la bande, pénalisante pour les bordures d'extrémité, et c'est pourquoi le déchirement est réalisé selon un mode axial. Le dispositif est donc conçu pour que ce déchirement axial puisse être mis en oeuvre.

A cet effet, un couple de mâchoires bloque la bande monocouche. Un autre couple de mâchoires s'écarte, selon l'axe de défilement, dudit premier couple, aux fins de réaliser ledit déchirement.

Cette solution ne peut pas s'appliquer à un matelas multicouches, dont chaque couple est au contact d'au moins une autre d'une manière lâche, avec des zones de repli plus solides etc... Le dispositif divulgué est donc de conception totalement inadéquate pour ce qui concerne le problème technique posé dans le présent brevet.

Comme on l'a dit, le type de sectionnement du dispositif de l'invention est obtenu par des moyens d'arrachage s'appliquant au tronçon situé en aval dudit dispositif, et opérant perpendiculairement à l'axe de défilement par déchirement du matériau se présentant initialement en bande continue. Lesdits moyens d'arrachage comportent des moyens de maintenir fermement ledit matériau, disposés de part et d'autre de la zone d'arrachage, pendant que des moyens de déchirement s'exercent sur lui pour sectionner le tronçon en aval.

Bien entendu, ces moyens n'interviennent pas simultanément, mais au moins partiellement séquentiellement, pour que le déchirement ne s'applique que lorsque la bande est fermement maintenue. Selon l'invention, le dispositif de sectionnement comporte par conséquent des moyens pour générer répétitivement et au moins partiellement séquentiellement le mouvement des moyens de maintien en position fixe dudit matériau en bande, et le mouvement des moyens de déchirement.

De préférence, lesdits moyens permettant de générer lesdits mouvements comportent au moins un premier embiellage reliant des moyens d'entraînement auxdits moyens de maintien immobile du matériau en bande, et au moins un second embiellage reliant des moyens d'entraînement aux moyens de déchirement, ledit moyen d'entraînement communiquant un mouvement alternatif auxdits moyens de maintien immobile et auxdits moyens de déchirement.

Selon une configuration possible, le moyen d'entraînement consiste dans les deux cas en un disque rotatif entraîné par un moyen moteur, à la périphérie duquel est reliée l'extrémité d'un bras de chaque embiellage. Ce disque est de préférence unique pour les deux embiellages, lesquels y sont au surplus fixés par une liaison à pivotement unique située sur la périphérie dudit disque. Ce dernier est préférentiellement entraîné par un moteur électrique, par exemple un moto-réducteur.

Dans le système de l'invention, le premier embiellage est en fait une genouillère composée de trois bras articulés à une de leurs extrémités autour d'un axe central de pivotement, les autres extrémités desdits bras pivotant respectivement sur le disque d'entraînement, sur un tourillon fixé au bâti auquel est fixé le dispositif de sectionnement, et sur un tourillon relié aux moyens de maintien immobile du matériau en bande.

Le second embiellage comprend deux bras, l'un ayant sa première extrémité pivotant sur le disque d'entraînement et sa seconde extrémité pivotant sur un tourillon fixé sur l'une des extrémités du second bras. Ce dernier pivote sur le bâti auquel est fixé le dispositif de séparation par l'intermédiaire d'un tourillon. La seconde de ses extrémités pivote autour d'un tourillon fixé aux moyens de déchirement.

Lorsqu'ils sont actionnés par les moyens d'entraînement évoqués auparavant, ces deux embiellages permettent d'animer au moins partiellement séquentiellement les moyens de maintien et le moyen de déchirement transversal de la bande.

Les moyens de maintien sont placés de part et d'autre de la bande, et se font face par rapport à un plan de défilement contenant l'axe de défilement et sensiblement médian par rapport à la bande. De préférence, les moyens de maintien situés d'un côté du matériau sont mobiles, alors que ceux qui sont placés en face sont fixes.

Par ailleurs, les moyens de maintien mobiles sont guidés linéairement dans un plan perpendiculaire à l'axe de défilement, par des glissières dans lesquelles lesdits moyens de maintien mobiles coulissent alternativement dans un sens et dans l'autre, pour s'approcher, entrer en contact et s'écarter des moyens de maintien fixes situés de l'autre côté du matériau en bande.

Lorsque lesdits moyens sont au contact, à travers le matériau qu'ils maintiennent par conséquent fermement, le déchirement peut avoir lieu. Toutefois, l'endroit exact du sectionnement n'est pas sans importance, et doit être localisé dans une zone de relativement petite dimension où la tension longitudinale due au système de maintien est la plus forte. De plus, l'action de déchirement doit s'exercer perpendiculairement à l'axe de défilement.

C'est pourquoi, les moyens de déchirement sont également guidés linéairement dans un plan perpendiculaire à l'axe de défilement, dans des glissières dans lesquelles ils coulissent alternativement dans un sens et dans l'autre, de manière à rencontrer la bande et à amorcer le déchirement ultérieur, par arrachement.

Bien entendu, les mouvements linéaires alternatifs des moyens de maintien immobile du matériau en bande et des moyens de déchirement sont réalisés respectivement à l'aide du premier et du second embiellage.

Les moyens de maintien cités sont de préférence des mors, au nombre de deux de chaque côté de la bande à maintenir, et séparés par un intervalle dans lequel se situent les moyens de déchirement.

Comme cela sera explicité en détail dans la suite du texte, il y a une interaction profonde entre les mors de maintien, comprenant de chaque côté deux surfaces transversales destinées à entrer au contact de la bande à sectionner en le comprimant, et les moyens de déchirement qui agissent entre les paires de mors.

Lesdits moyens de déchirement consistent en au moins une plaque conçue pour provoquer un déchirement perpendiculairement à l'axe de défilement lorsque les mors placés de part et d'autre de la bande de matériau sont au contact les uns des autres via la bande et l'immobilisent.

De préférence, cette plaque de déchirement est du type scie à dents. Au cours du mouvement de la plaque vers la bande de matériau fermement maintenue par les mors, les dents pointues rencontrent la portion tendue entre lesdits mors, et opèrent une perforation initiale qui s'élargit au fur et à mesure que l'interaction bande/plaque augmente : le matériau en bande est alors sectionné après affaiblissement progressif le long d'une ligne transversale, qui se termine par l'arrachage au moment où les perforations se rejoignent.

Selon une configuration préférentielle, les mors fixes constituent au moins partiellement la glissière de guidage de la lame de déchirement, laquelle se déplace entre eux et également entre les mors mobiles au cours de la phase de déchirement. Cette solution présente l'avantage supplémentaire de garantir le positionnement de la plaque en relation transversale avec la zone maintenue immobile et tendue de la bande à sectionner.

Au cours du fonctionnement, selon une possibilité, les mors mobiles et la plaque de déchirement se déplacent devant une ouverture laissant passer le matériau en bande, la longueur du déplacement étant au moins égale à la largeur de ladite ouverture. Comme on l'a dit, un tel dispositif de séparation ou sectionnement pourra être utilisé en combinaison avec différents systèmes fournissant un matériau en bande devant être tronçonné. Ainsi, une telle ouverture sera par exemple l'ouverture de sortie d'un tunnel d'amenée de la bande, situé en aval de sections appliquant d'autres traitements à ladite bande.

Le fait que la longueur du déplacement des mors mobiles et de la plaque de déchirement soit supérieure à la largeur de ladite ouverture évite tout dysfonctionnement en sortie, par exemple évite que le tronçon sectionné reste au contact des mors mobiles ou de la lèvre de déchirement. Ceci sera expliqué plus en détail ci-après.

Une machine selon l'invention peut comporter une fenêtre de sortie d'allure rectangulaire située en amont du dispositif, et par laquelle passe le matériau en bande avant arrachement des tronçons successifs.

Le dispositif est de préférence fixé sur un panneau dans lequel est pratiquée ladite ouverture, de manière que les moyens mobiles de maintien du matériau en bande et les moyens de déchirement, à savoir respectivement les mors mobiles et la plaque de déchirement, coulissent devant ladite ouverture. Ce panneau peut être partie intégrante du bâti de la machine, ou rapporté sur ce dernier.

Comme on l'a vu, l'avantage majeur du produit obtenu par rapport à ses devanciers est d'être beaucoup plus cohérent, c'est à dire doté d'un haut pouvoir de maintien des différentes couches au contact les unes des autres. Le déchirement aux deux extrémités, du fait des circonstances de son obtention, produit une sorte d'agrafage des sections d'extrémités, dû aux irrégularités de la zone de sectionnement et au pliage desdites irrégularités, conduisant à une adhésion mutuelle des couches dans ces zones.

On va maintenant décrire plus en détail l'invention objet du présent brevet, en se référant aux figures annexées, pour lesquelles :
- La figure 1 est une vue en plan du dispositif de séparation selon la présente invention ;
- La figure 2 est une vue de côté du même dispositif ;
- Les figures 3a à 3d montrent des vues équivalentes en élévation des différentes phases successives du processus d'arrachement de tronçons sur une bande,
- La figure 4 est une vue en section selon la ligne IV-IV de la figure 1, dans la direction des flèches,
- La figure 5 est un graphique montrant la cinématique du dispositif, c'est à dire faisant ressortir la position relative des moyens de maintien et des moyens d'arrachement dans les phases illustrées aux figures 3a-3d.

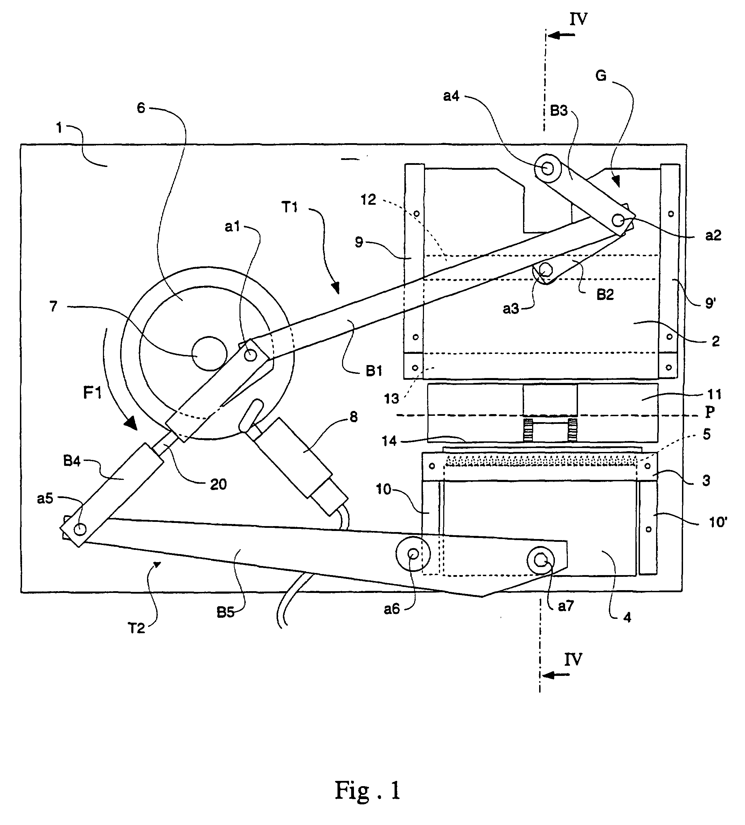

Le dispositif de l'invention est de préférence fixé à un bâti (1), soit directement à un carter de machine, soit préférentiellement à un panneau fixable par tout moyen approprié audit carter. Dans la configuration préférentielle décrite ci-dessous, le dispositif de séparation comporte essentiellement des mors mobiles (2, 2'), des mors fixes (3, 3') et une plaque de déchirement (4), du type en lame de scie à dents (5), apparaissant notamment en figure 1. La bande de matériau progresse suivant l'axe de défilement (A) et sensiblement dans le plan médian (P).

Les mors mobiles (2, 2') sont reliés à un premier embiellage (T1) comportant notamment une genouillère (G), alors que la plaque de déchirement (4) est reliée à un second embiellage (T2), lesdits embiellages (T1, T2) communiquant aux parties mobiles (mors mobiles (2, 2') et plaque (4)) un mouvement provenant d'un disque d'entraînement (6). Ce dernier est lui-même entraîné par un moyen moteur (non représenté) via l'arbre (7), ledit moyen moteur étant de préférence un moto-réducteur électrique.

Un moto-réducteur électrique offre en effet la souplesse de commande nécessaire à une automatisation poussée de l'opération de séparation par arrachement, dont les interventions peuvent être répétitives, à fréquence variable, ou isolées. Le disque d'entraînement (6) est d'ailleurs sectionné selon une de ses cordes, pour permettre l'interaction avec un capteur (8) de position nécessaire à la fourniture d'informations concernant les rotations dudit disque, et donc aussi le fonctionnement des organes d'arrachage.

L'embiellage (T1) communique le mouvement du disque (6) aux mors mobiles (2, 2'), la transformation du mouvement rotatif en un mouvement translatif étant notamment réalisée à l'aide des glissières parallèles (9, 9') fixées sur le bâti (1). L'ensemble comporte quatre axes de pivotement correspondant à chaque fois à un tourillon (a1, a2, a3, a4) autour duquel a lieu la rotation.

Le tourillon (a1) est fixé sur une zone périphérique du disque (6), de manière à produire un mouvement circulaire de rayon égal à la distance entre l'axe (7) et le tourillon (a1). Le tourillon (a2) n'est pas relié aux mors mobiles (2, 2'), mais uniquement aux trois bras (B1, B2, B3) constituant la genouillère (G). Le tourillon (a3) permet le pivotement de (B2) sur lesdits mors mobiles (2, 2'), alors que le tourillon (a4) est fixé au bâti (1), et constitue l'axe fixe de la genouillère (G). Ce système permet de communiquer aux mors mobiles (2, 2') un mouvement rectiligne alternatif en direction des mors fixes (3), avec une force de contact suffisante pour maintenir fermement la bande de matériau pendant la séparation par arrachement de tronçons, la direction précise du mouvement étant donnée par les glissières (9, 9').

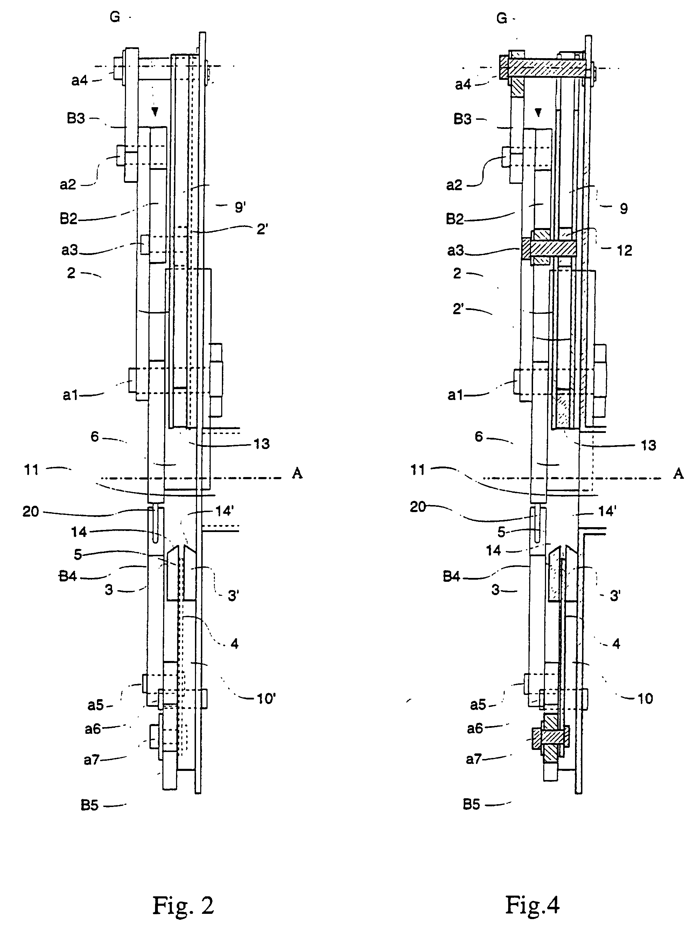

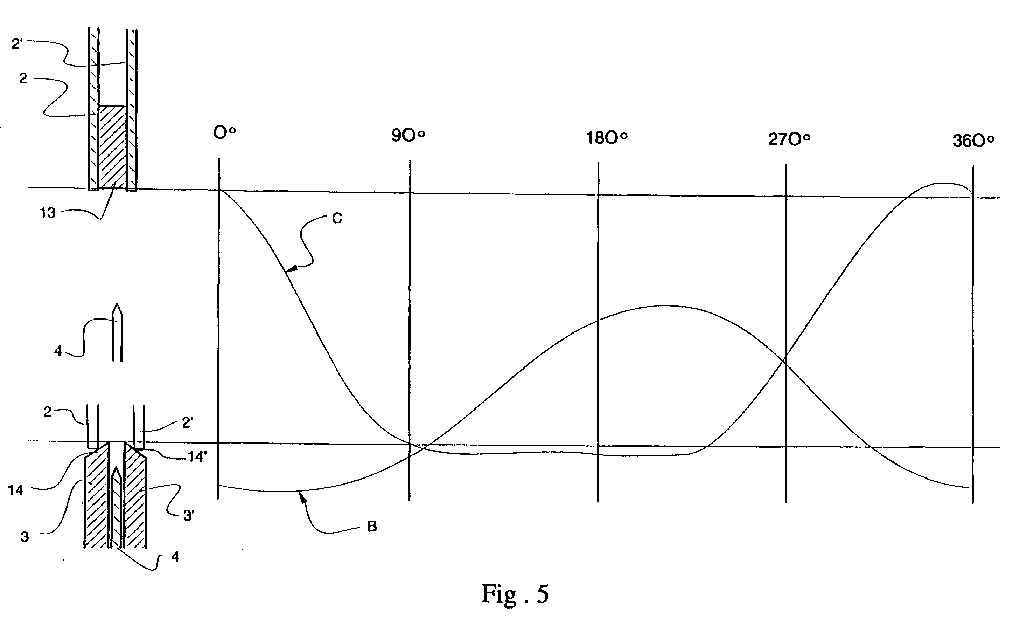

On notera que la position du tourillon (a4) est réglable par rapport au bâti (1), au moyen d'un excentrique apparaissant notamment en figures 2 et 4, selon un montage connu en soi.

L'embiellage (T2) fonctionne suivant un principe similaire : les tourillons de pivotement supplémentaires sont référencés (a5, a6, a7) et contrôlent le mouvement des bras (B4, B5), imprimant également un mouvement rectiligne alternatif à la lame de déchirement (4) avec l'aide des glissières (10, 10'). Le pivotement au niveau du disque d'entraînement (6) a lieu autour du même tourillon (a1) que précédemment. Le tourillon (a5) n'est pas relié au bâti (1), à l'inverse du tourillon (a6) servant d'axe de pivotement fixe pour le bras (B5). Enfin, le tourillon (a7) relie ledit bras (B5) à la plaque de déchirement (4), et lui communique finalement le mouvement initié au niveau du disque (6).

Comme on le verra dans la suite, ces deux embiellages (T1, T2) aboutissent à communiquer aux mors mobiles (2, 2') et à la plaque (4) un mouvement séquentiel, la plaque agissant bien évidemment après les mors. Les deux glissières de coulissement (9, 9', 10, 10') sont disposées de part et d'autre d'une ouverture rectangulaire (11) laissant apparaître un dispositif central d'entraînement et de froissage d'une machine à produire du matériau de calage/rembourrage, ainsi que le plan moyen de défilement (P).

Les embiellages sont susceptibles de réglage, comme notamment l'ajustement de la longueur du bras (B4), par exemple au moyen d'une tige filetée (20). Il est également à noter que la plaque de déchirement (4) a une largeur inférieure à la distance séparant les deux glissières (10, 10'). De la sorte, il y a une amorce de pivotement qui s'opère, permettant à ladite plaque (4) d'attaquer la bande légèrement de biais, ce qui améliore l'effet de séparation.

Les figures 2 et 4 permettent d'avoir un autre éclairage du dispositif de l'invention, en vue de côté et en coupe selon la ligne IV-IV. Ces figures montrent clairement un détail essentiel de l'invention : il y a deux paires de mors (2, 2', 3, 3'), et la lame de scie (4) se déplace à l'intérieur de l'espace résiduel existant entre chaque paire. Ceci est très important, car ladite lame (4) agit de fait dans la zone de tension et de maintien maximum résultant de l'application des mors (2, 2', 3, 3') sur la bande de papier.

La paire de mors mobiles comporte deux plaques parallèles (2, 2') reliées par une entretoise (12) au niveau du tourillon (a3), laissant notamment libre l'espace résiduel entre elles à proximité de l'ouverture rectangulaire (11). L'un des moyens de guidage additionnels consiste en une entretoise (13) située à proximité de cette même ouverture (11).

La paire de mors fixes se compose également de deux plaques (3, 3') de plus petite dimension, espacées d'une fente suffisante pour laisser le passage à la plaque de déchirement (4). La partie destinée à venir au contact des mors mobiles (2, 2') comprend une extrémité supérieure fraisée en biais (14, 14') pour permettre un certain débattement des plaques sous l'effet de leur compression.

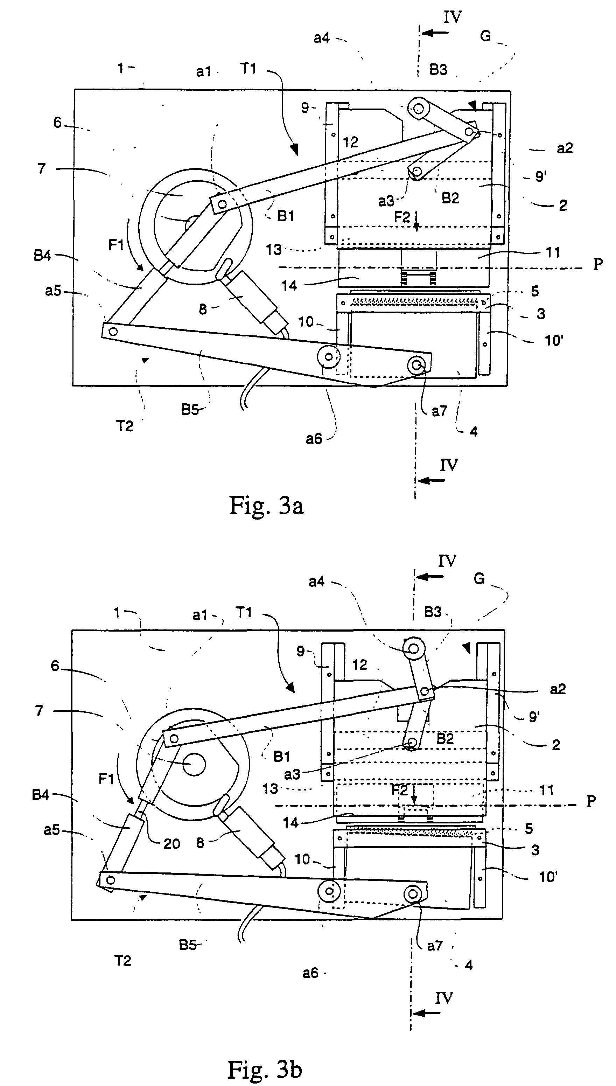

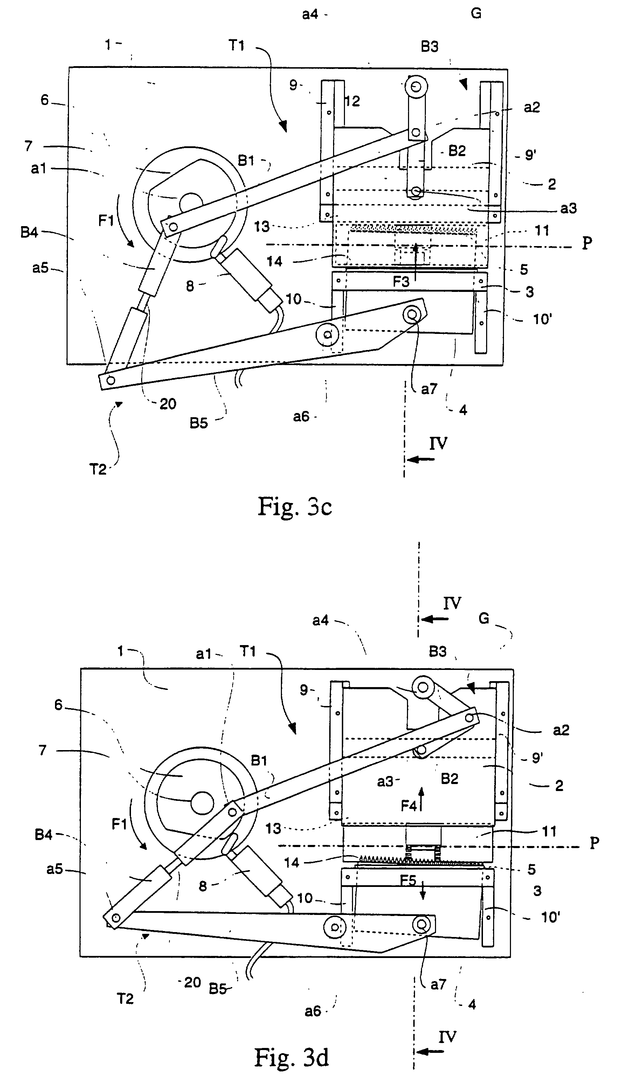

En se référant maintenant aux figures 3a à 3d et 5, on observe un cycle de fonctionnement complet, débutant pratiquement en figure 3a et s'achevant sensiblement à la figure 3d. Dans un premier temps, le plateau (6) tournant dans le sens de la flèche (F1), les mors mobiles (2, 2') sont mis en mouvement dans le sens de la flèche (F2), c'est à dire vers les mors fixes (3, 3'), en passant devant l'ouverture (11), jusqu'à atteindre les parties inclinées (14, 14') des mors fixes (3, 3') via la bande à sectionner qui laissera en pratique subsister une très petite distance entre les deux paires de mors.

Pendant cette première phase, la plaque de déchirement mobile (4) ne bouge pratiquement pas, si ne n'est un léger pivotement initial accompagnant le début du mouvement rectiligne vers la bande. C'est ce qui apparaît en figure 3b.

Ensuite, les mors mobiles (2, 2') restent en pression contre les mors fixes (3, 3') en immobilisant ladite bande, pendant que la plaque (4) remonte entre les mors mobiles (2, 2'), comme montré par la flèche (F3) en figure 3c. Il est à noter que la pression exercée par lesdits mors mobiles (2, 2') sur les mors fixes (3, 3') n'est pas constante, mais passe par un maximum coïncidant sensiblement avec le paroxysme de la phase de séparation. Le travail des dents (5) de la plaque (4) s'exerce très progressivement, la séparation finale entraînant la contrainte de sectionnement la plus élevée ayant lieu au moment ou le serrage des mors est maximal, impliquant des tensions longitudinales suffisantes pour équilibrer les contraintes de sectionnement contraires.

Les mors mobiles (2, 2') et la plaque (4) se retirent ensuite dans le sens des flèches (F4, F5) (figure 3d) selon des trajets qui vont au-delà de l'entretoise (13) pour les mors mobiles (2, 2') et au-delà des mors fixes (3, 3') pour la lame (4). Cette dernière caractéristique a pour objet de décoller une couche de papier qui resterait éventuellement collée soit aux mors mobiles (2, 2'), soit à la lame (4).

Les trajets respectifs des mors mobiles (2, 2') et de la plaque d'arrachement et de séparation (4) apparaissent clairement dans leur relation mutuelle sur le graphique de la figure 5 sur lequel la courbe (C) illustre le trajet des mors (2, 2') et la courbe (B) illustre le trajet de la plaque dentée (4) au cours d'un cycle complet du dispositif.

On a figuré en traits plus fins les positions extrêmes des mors mobiles (2, 2') et de la plaque dentée (4). La courbe (C) présente un minimum aplati traduisant la durée pendant laquelle les deux paires de mors (2, 2', 3, 3') sont au contact (un peu moins d'une demi-période).

La courbe (B) présente l'allure d'une quasi sinusoïde, traduisant la régularité du mouvement de la plaque (4), avec un maximum qui intervient bien entendu pendant le serrage des mors (2, 2', 3, 3').

Le maximum de la courbe (C), placé en fin de cycle et par conséquent juste avant un nouveau cycle, illustre la caractéristique énoncée précédemment : les mors mobiles (2, 2') vont au-delà de l'entretoise (13) pour éventuellement décoller une couche de papier qui resterait au contact desdits mors mobiles (2, 2').

Il en va de même pour le minimum de la courbe B, qui est situé bien en deçà des mors fixes (3, 3'), pour la même raison, mais appliquée à la plaque (4).

Le matériau en bande défilant selon l'axe (A) et tronçonné par arrachement à ses deux extrémités, a une configuration très particulière du type en coussinet dont les couches sont aplaties et entremêlées auxdites extrémités, du fait de la nature particulière du sectionnement.

La machine et le procédé selon la présente invention permettent par conséquent d'aboutir à un produit dont la cohésion est très supérieure à celle des produits tronçonnés par coupe à lames traditionnelles, opérant selon un mode de fonctionnement de type cisaille.

## Revendications

1. Machine pour la fabrication d'un matériau de calage/rembourrage à partir d'un matériau en bande continue de type papier comprenant au moins une couche, par repli longitudinal des bordures latérales de la bande vers la zone centrale de ladite bande, de manière qu'il y ait superposition desdites bordures dans ladite zone centrale, puis par entraînement de l'ensemble replié, froissage de l'ensemble replié et enfin assemblage de l'ensemble froissé par des compressions ponctuelles successives, **caractérisée en ce qu'**elle comprend en sortie un dispositif de séparation par arrachement de tronçons successifs comportant des moyens d'arrachage du tronçon situé en aval dudit dispositif de séparation, ces moyens d'arrachage opérant perpendiculairement a l'axe de défilement (A) de la bande par déchirement de ladite bande matelassée, et comportant des moyens (2, 2', 3, 3') pour maintenir fermement le matériau en bande dans une position immobile au moins dans la zone d'arrachage, disposés de part et d'autre des moyens de déchirement (4) qui s'exercent sur ledit matériau en bande por séparer le tronçon situé en aval dudit dispositif de séparation.

2. Machine selon la revendication 1, **caractérisé en ce que** le dispositif comprend des moyens pour générer répétitivement et au moins partiellement séquentiellement le mouvement des moyens (2, 3) de maintien en position fixe du matériau en bande et le mouvement des moyens de déchirement (4).

3. Machine selon la revendication 2, **caractérisé en ce que** lesdits moyens permettant de générer lesdits mouvements comportent au moins un premier embiellage (T1) reliant un moyen d'entraînement (6, 7) aux dits moyens (2, 3) de maintien en position immobile du matériau en bande, et au moins un second embiellage (T2) reliant ledit moyen d'entraînement (6, 7) aux moyens de déchirement (4), lesdits moyens d'entraînement communs (6, 7) leur communiquant un mouvement alternatif.

4. Machine selon la revendication 3, **caractérisé en ce que** lesdits moyens d'entraînement communs consistent en un disque rotatif (6) entraîné par un moyen moteur (7) et à la périphérie duquel est reliée l'extrémité d'un bras (B1, B4) de chaque embiellage (T1, T2).

5. Machine selon la revendication 4, **caractérisé en ce que** le disque (6) étant unique pour les deux embiellages (T1, T2), chacun des bras (B1, B4) est entraîné par ce disque par un tourillon unique (a1) fixé sur la périphérie dudit disque (6), ce dernier étant entraîné par un moto-réducteur électrique.

6. Machine selon l'une des revendications 3 à 5, **caractérisé en ce que** le premier embiellage (T1) est une genouillère (G) composée de trois bras (B1, B2, B3) articulés à une de leurs extrémités autour d'un tourillon unique (a2), les autres extrémités desdits bras (B1, B2, B3) pivotant respectivement sur le tourillon (a1) du disque d'entraînement (6), sur un tourillon (a4) fixé à un bâti (1) de la machine à laquelle est adapté le dispositif de sectionnement, et sur un tourillon (a3) fixé aux moyens mobiles (2, 2') de maintien immobile du matériau en bande.

7. Machine selon l'une des revendications 3 à 6, **caractérisé en ce que** le second embiellage (T2) comprend deux bras (B4, B5), l'un (B4) ayant sa première extrémité pivotant sur le tourillon (a1) du disque d'entraînement (6) et sa seconde extrémité pivotant sur un tourillon (a5) fixé à l'une des extrémités du second bras (B5), ledit second bras (B5) étant monté basculant autour d'un tourillon (a6) fixé sur le bâti (1) et étant articulé, à l'une de ses extrémités sur ledit tourillon (a5) et à l'autre extrémité sur un tourillon (a7) fixé aux moyens de déchirement (4).

8. Machine selon l'une des revendications 1 à 7, **caractérisé en ce que** les moyens (2, 3) de maintien immobile du matériau en bande sont placés de part et d'autre de ladite bande, et se font face par rapport au plan moyen de défilement de cette bande.

9. Machine selon la revendication 8, **caractérisé en ce que** les moyens (2, 2') de maintien situés d'un premier côté du matériau en bande sont mobiles, alors que les moyens de maintien situés de l'autre côté (3, 3') sont fixes.

10. Machine selon la revendication 9, **caractérisé en ce que** les moyens de maintien mobiles (2, 2') sont guidés linéairement dans un plan perpendiculaire à l'axe de défilement (A), par des glissières (9, 9') dans lesquelles lesdits moyens de maintien mobiles (2, 2') coulissent alternativement dans un sens et dans l'autre, pour s'approcher, entrer en contact et s'écarter des moyens de maintien fixes (3, 3') situés de l'autre côté du matériau en bande.

11. Machine selon l'une quelconque des revendications 1 à 10, **caractérisé en ce que** les moyens de déchirement (4) sont guidés linéairement dans un plan perpendiculaire à l'axe de défilement (A), dans des glissières (10, 10') dans lesquelles ils coulissent alternativement dans un sens et dans l'autre.

12. Machine selon l'une des revendications 10 et 11, **caractérisé en ce que** les mouvements linéaires alternatifs des moyens de maintien mobiles (2, 2') du matériau en bande et des moyens de déchirement (4) sont commandés respectivement par le premier embiellage (T1) et par le second embiellage (T2).

13. Machine selon l'une des revendications 1 à 12, **caractérisé en ce que** les moyens (2, 3) de maintien de la bande en position immobile sont des mors (2, 2', 3, 3'), au nombre de deux de chaque côté de la bande de matériau à sectionner, et séparés par un intervalle dans lequel sont situés et se déplacent les moyens de déchirement (4).

14. Machine selon l'une des revendications 1 à 13, **caractérisé en ce que** lesdits moyens de déchirement consistent en au moins une plaque (4) dont un côté comporte des dents (5) opérant un déchirement perpendiculairement à l'axe de défilement lorsque les mors (2, 2', 3, 3') placés de part et d'autre de la bande de matériau sont au contact les uns des autres via la bande pour la maintenir fixe.

15. Machine selon la revendication 14, **caractérisé en ce que** la plaque de déchirement (4) est du type lame de scie à dents.

16. Machine selon l'une des revendications 13 à 15, **caractérisé en ce que** les moyens de maintien fixes (3, 3') constituent au moins partiellement la glissière de guidage des moyens de déchirement (4), lesquels se déplacent entre eux et également entre les mors mobiles (2, 2') au cours de la phase de déchirement.

17. Machine selon l'une des revendications 14 à 16, **caractérisé en ce que** les mors mobiles (2, 2') et la lame de déchirement (4) se déplacent devant une ouverture (11) laissant passer le matériau en bande à sectionner, la longueur de leur déplacement étant au moins égale à la largeur de ladite ouverture.

18. Machine pour la fabrication d'un matériau de calage/rembourrage selon la revendication 18, **caractérisée en ce qu'**elle comporte une fenêtre de sortie (11) d'allure rectangulaire située en amont du dispositif de séparation, et par laquelle passe le matériau en bande.

19. Machine pour la fabrication d'un matériau de calage/rembourrage selon la revendication 1, **caractérisée en ce que** le dispositif de séparation est fixé sur un panneau (1) dans lequel est pratiquée ladite ouverture (11), de manière que les moyens mobiles (2, 2') de maintien du matériau en bande et les moyens de déchirement (4) coulissent devant ladite ouverture (11) dans des plans parallèles au plan du panneau (1).

20. Procédé d'obtention de matériau de calage/rembourrage à partir d'un matériau en bande de type papier comprenant au moins une couche, comprenant les étapes suivantes :
- repli longitudinal des bordures latérales de la bande vers la zone centrale de ladite bande,
- entraînement de l'ensemble replié,
- froissage de l'ensemble replié, et enfin
- assemblage de l'ensemble froissé par des compressions ponctuelles successives,
**caractérisé en ce que** l'étape finale consiste à opérer un tronçonnage dudit matériau à l'aide d'un dispositif de séparation muni de moyens d'arrachage du tronçon situé en aval dudit dispositif, le dispositif comportant des moyens d'arrachage du tronçon situé en aval dudit dispositif de séparation, ces moyens d'arrachage opérant perpendiculairement audit axe de défilement (A) par déchirement de ladite bande matelassée, et comportant des moyens (2, 2', 3, 3') pour maintenir fermement le matériau en bande dans une position immobile au moins dans la zone d'arrachage, disposés de part et d'autre des moyens de déchirement (4) qui s'exercent sur ledit matériau en bande pour séparer le tronçon situé en aval dudit dispositif de séparation, la bande s'avançant suivant un axe de défilement (A) perpendiculaire audit dispositif.

## Patentansprüche

1. Maschine für die Herstellung eines Verkeilungs-/Polsterungsmaterials aus einem wenigstens einschichtigen, ununterbrochenen Bandmaterial des Typs Papier durch longitudinales Umfalten der seitlichen Ränder des Bandes zur Mittelzone des Bandes, so daß die Ränder in der Mittelzone überlagert sind, dann durch Antreiben der umgefalteten Gesamtheit, Zerknittern der umgefalteten Gesamtheit und schließlich Zusammenfügen der zerknitterten Gesamtheit durch aufeinanderfolgende punktförmige Kompressionen,
**dadurch gekennzeichnet,**
**daß** sie am Auslaß eine Trennvorrichtung durch Abreißen aufeinanderfolgender Teilstücke, Mittel zum Abreißen eines Teilstücks, das sich hinter der Trennvorrichtung befindet, wobei diese Abreißmittel durch Durchreißen des Polsterungsbandes senkrecht zur Vorbeibewegungsachse (A) des Bandes arbeiten, und Mittel (2, 2', 3, 3') zum Festhalten des Bandmaterials in einer unbeweglichen Stellung wenigstens in der Abreißzone, die beiderseits von Durchreißmitteln (4) angeordnet sind, die auf das Bandmaterial wirken, um das hinter der Trennvorrichtung befindliche Teilstück abzutrennen, umfaßt.

2. Maschine nach Anspruch 1,
**dadurch gekennzeichnet,**
**daß** sie Mittel zum wiederholten und wenigstens teilweise sequentiellen Erzeugen der Bewegung der Mittel (2, 3) zum Halten des Bandmaterials in der unbeweglichen Stellung und der Bewegung der Durchreißmittel (4) umfaßt.

3. Maschine nach Anspruch 2,
**dadurch gekennzeichnet,**
**daß** die Mittel, die die Erzeugung der Bewegungen ermöglichen, wenigstens ein erstes Gestänge (T1), das ein Antriebsmittel (6, 7) mit den Mitteln (2, 3) zum Halten des Bandmaterials in der unbeweglichen Stellung verbindet, und wenigstens ein zweites Gestänge (T2), das die Antriebsmittel (6, 7) mit den Durchreißmitteln (4) verbindet, umfassen, wobei die gemeinsamen Antriebsmittel (6, 7) an sie eine Hin- und Herbewegung übertragen.

4. Maschine nach Anspruch 3,
**dadurch gekennzeichnet,**
**daß** die gemeinsamen Antriebsmittel aus einer rotierenden Scheibe (6) bestehen, die durch ein Motormittel (7) angetrieben wird und mit deren Umfang das Ende eines Arms (B1, B4) jedes Gestänges (T1, T2) verbunden ist.

5. Maschine nach Anspruch 4,
**dadurch gekennzeichnet,**
**daß**, da die Scheibe (6) den beiden Gestängen (T1, T2) gemeinsam ist, jeder der Arme (B1, B4) durch diese Scheibe über einen einzigen Drehzapfen (a1) angetrieben wird, der am Umfang der Scheibe (6) befestigt ist, wobei diese letztere durch einen elektrischen Getriebemotor angetrieben wird.

6. Maschine nach einem der Ansprüche 3 bis 5,
**dadurch gekennzeichnet,**
**daß** das erste Gestänge (T1) ein Kniehebel (G) ist, der aus drei Armen (B1, B2, B3) gebildet ist, die mit einem ihrer Enden an einem einzigen Drehzapfen (a2) angelenkt sind, während die anderen Enden der Arme (B1, B2, B3) am Drehzapfen (a1) der Antriebsscheibe (6) bzw. an einem Drehzapfen (a4), der am Rahmen (1) der Maschine, für die die Unterteilungsvorrichtung bestimmt ist, befestigt ist, bzw. an einem Drehzapfen (a3), der an beweglichen Mitteln (2, 2') zum Festhalten des Bandmaterials befestigt ist, angelenkt sind.

7. Maschine nach einem der Ansprüche 3 bis 6,
**dadurch gekennzeichnet,**
**daß** das zweite Gestänge (T2) zwei Arme (B4, B5) umfaßt, wovon einer (B4) mit seinem ersten Ende am Drehzapfen (a1) der Antriebsscheibe (6) angelenkt ist und mit seinem zweiten Ende an einem Drehzapfen (a5) angelenkt ist, der an einem der Enden des zweiten Arms (B5) befestigt ist, wobei der zweite Arm (B5) an einem am Rahmen (1) befestigten Drehzapfen (a6) schwenkbar angebracht ist und an einem seiner Enden am Drehzapfen (a5) angelenkt und am anderen Ende an einem Drehzapfen (a7), der an den Durchreißmitteln (4) befestigt ist, angelenkt ist.

8. Maschine nach einem der Ansprüche 1 bis 7,
**dadurch gekennzeichnet,**
**daß** die Mittel (2, 3) zum Festhalten des Bandmaterials beiderseits des Bandes angeordnet sind und über eine Mittelebene der Vorbeibewegung dieses Bandes einander zugewandt sind.

9. Maschine nach Anspruch 8,
**dadurch gekennzeichnet,**
**daß** die Haltemittel (2, 2'), die sich auf einer ersten Seite des Bandmaterials befinden, beweglich sind, während die Haltemittel (3, 3'), die sich auf der anderen Seite befinden, fest sind.

10. Trennvorrichtung nach Anspruch 9,
**dadurch gekennzeichnet,**
**daß** die beweglichen Haltemittel (2, 2') geradlinig in einer Ebene senkrecht zur Vorbeibewegungsachse (A) durch Gleitschienen (9, 9') geführt werden, in denen die beweglichen Haltemittel (2, 2') hin und her gleiten, um sich den festen Haltemitteln (3, 3'), die sich auf der anderen Seite des Bandmaterials befinden, anzunähern, mit ihnen in Kontakt zu gelangen und sich von ihnen zu entfernen.

11. Maschine nach einem der Ansprüche 1 bis 11,
**dadurch gekennzeichnet,**
**daß** die Durchreißmittel (4) in einer Ebene senkrecht zur Vorbeibewegungsachse (A) in Gleitschienen (10, 10'), in denen sie hin und her gleiten, geradlinig geführt werden.

12. Maschine nach einem der Ansprüche 10 und 11,
**dadurch gekennzeichnet,**
**daß** die geradlinigen Hin- und Herbewegungen der beweglichen Haltemittel (2, 2') für das Bandmaterial und der Durchreißmittel (4) durch das erste Gestänge (T1) bzw. durch das zweite Gestänge (T2) gesteuert werden.

13. Maschine nach einem der Ansprüche 1 bis 12,
**dadurch gekennzeichnet,**
**daß** die Mittel (2, 3) zum Halten des Bandes in einer unbeweglichen Stellung Backen (2, 2', 3, 3') in der Anzahl zwei auf jeder Seite des zu unterteilenden Materialbandes sind, die durch einen Zwischenraum getrennt sind, in dem sich die Durchreißmittel (4) befinden und verschieben.

14. Maschine nach einem der Ansprüche 1 bis 13,
**dadurch gekennzeichnet,**
**daß** die Durchreißmittel aus wenigstens einer Platte (4) bestehen, wovon eine Seite Zähne (5) umfaßt, die ein Durchreißen senkrecht zur Vorbeibewegungsachse bewirken, wenn die beiderseits des Materialbandes angeordneten Backen (2, 2', 3, 3') miteinander über das Band in Kontakt sind, um es festzuhalten.

15. Maschine nach Anspruch 14,
**dadurch gekennzeichnet,**
**daß** die Durchreißplatte (4) vom Typ Sägezahnblatt ist.

16. Maschine nach einem der Ansprüche 13 bis 15,
**dadurch gekennzeichnet,**
**daß** die festen Haltemittel (3, 3') wenigstens teilweise die Gleitschiene zum Führen der Durchreißmittel (4) bilden, die sich zwischen ihnen und während der Durchreißphase außerdem zwischen den beweglichen Backen (2, 2') verschieben.

17. Maschine nach einem der Ansprüche 14 bis 16,
**dadurch gekennzeichnet,**
**daß** sich die beweglichen Backen (2, 2') und das Durchreißblatt (4) vor einer Öffnung (11) verschieben, die das zu unterteilende Bandmaterial durchläßt, wobei die Länge ihrer Verschiebung wenigstens gleich der Breite der Öffnung ist.

18. Maschine für die Herstellung eines Verkeilungs-/Polsterungsmaterials nach Anspruch 17,
**dadurch gekennzeichnet,**
**daß** sie ein Auslaßfenster (11) mit rechtwinkligem Verlauf besitzt, das sich vor der Trennvorrichtung befindet und das Bandmaterial durchläßt.

19. Maschine für die Herstellung eines Verkeilungs-/Polsterungsmaterials nach Anspruch 1,
**dadurch gekennzeichnet,**
**daß** die Trennvorrichtung an einer Platte (1) befestigt ist, in der die Öffnung (11) ausgebildet ist, so daß die beweglichen Mittel (2, 2') zum Halten des Bandmaterials und die Durchreißmittel (4) vor der Öffnung (11) in zur Ebene der Platte (1) parallelen Ebenen gleiten.

20. Verfahren zum Erhalten eines Verkellungs-/Poisterungsmaterials aus einem wenigstens einschichtigen Bandmaterial des Typs Papier, das die folgenden Schritte umfaßt:
- longitudinales Umfalten der seitlichen Ränder des Bandes zur Mittelzone des Bandes,
- Antreiben der umgefalteten Gesamtheit,
- Zerknittern der umgefalteten Gesamtheit und schließlich
- Zusammenfügen der zerknitterten Gesamtheit durch aufeinanderfolgende punktförmige Kompressionen,
**dadurch gekennzeichnet,**
**daß** der abschließende Schritt darin besteht, eine Zerteilung des Materials in Teilstücke mit Hilfe einer Trennvorrichtung vorzunehmen, die mit Mitteln zum Abreißen eines hinter der Vorrichtung befindlichen Teilstücks versehen ist, wobei die Vorrichtung Mittel zum Abreißen eines Teilstücks, das sich hinter der Trennvorrichtung befindet, wobei diese Abreißmittel durch Durchreißen des Polsterungsbandes senkrecht zur Vorbeibewegungsachse (A) arbeiten, und Mittel (2, 2', 3, 3') zum Festhalten des Bandmaterials in einer unbeweglichen Stellung wenigstens in der Abreißzone, die beiderseits von Durchreißmitteln (4) angeordnet sind, die auf das Bandmaterial wirken, um das hinter der Trennvorrichtung befindliche Teilstück abzutrennen, umfaßt, wobei sich das Band längs einer Vorbeibewegungsachse (A) senkrecht zur Vorrichtung vorwärtsbewegt.

## Claims

1. Machine for production of a wedging/padding material from a material in a continuous strip of the paper type comprising at least one layer, by longitudinal folding of the lateral edges of the strip towards the central area of the said strip, such that there is superimposition of the said edges in the said central area, then by driving the folded assembly, crumpling the folded assembly and finally assembling the crumpled assembly by successive selective compressions, **characterised in that** it comprises at its output a device for separation by tearing of successive sections comprising means for tearing the section situated downstream from the said device for separation, these means for tearing operating perpendicularly to the axis of progression (A) of the strip by tearing of the said padded strip, and comprising means (2,2',3,3') for keeping the material in the form of a strip firmly in an immobile position at least in the area of tearing, which means are disposed on both sides of the means (4) for tearing which are applied to the said material in the form of a strip in order to separate the section situated downstream from the said device for separation.

2. Machine according to claim 1, **characterised in that** the device comprises means for generating repetitively and at least partially sequentially the movement of the means (2,3) for keeping the material in the form of a strip in a fixed position and the movement of the means (4) for tearing.

3. Machine according to claim 2, **characterised in that** the said means which make it possible to generate the said movement comprise at least one first link mechanism (T1) which connects a means (6,7) for driving to the said means (2,3) for keeping the material in the form of a strip in an immobile position, and at least a second link mechanism (T2) which connects the said means (6,7) for driving to the means (4) for tearing, the said common means (6,7) for driving imparting to the latter alternating motion.

4. Machine according to claim 3, **characterised in that** the said common means for driving consist of a rotary disc (6) which is driven by a drive means (7) and to the periphery of which there is connected the end of an arm (B1,B4) of each link mechanism (T1,T2).

5. Machine according to claim 4, **characterised in that** since there is a single disc (6) for the two link mechanisms (T1,T2), each of the arms (B1,B4) is driven by this disc by means of a single journal (a1) which is secured to the periphery of the said disc (6), the latter being driven by an electric gear motor.

6. Machine according to any one of claims 3 to 5, **characterised in that** the first link mechanism (T1) is a toggle joint (G) consisting of three arms (B1,B2,B3) which are articulated at one of their ends around a single journal (a2), the other ends of the said arms (B1,B2,B3) pivoting respectively on the journal (a1) of the drive disc (6), on a journal (a4) which is secured to a frame (1) of the machine on which the device for dividing up is fitted, and on a journal (a3) which is secured to the mobile means (2,2') for keeping the material in the form of a strip immobile.

7. Machine according to any one of claims 3 to 6, **characterised in that** the second link mechanism (T2) comprises two arms (B4,B5), one of them (B4) having its first end which pivots on the journal (a1) of the drive disc (6) and its second end which pivots on a journal (a5) which is secured to one of the ends of the second arm (B5), the said second arm (B5) being fitted such as to tilt around a journal (a6) which is secured to the frame (1) and is articulated at one of its ends on the said journal (a5) and at the other end on a journal (a7) which is secured to the means (4) for tearing (4).

8. Machine according to any one of claims 1 to 7, **characterised in that** the means (2,3) for keeping the material in the form of a strip immobile are placed on both sides of the said strip, and face one another relative to the mean plane of progression of this strip.

9. Machine according to claim 8, **characterised in that** the means (2,2') for retention which are situated on a first side of the material in the form of a strip are mobile, whereas the means (3,3') for retention which are situated on the other side are fixed.

10. Machine according to claim 9, **characterised in that** the mobile means (2,2') for retention are guided linearly on a plane which is perpendicular to the axis of progression (A) by slides (9,9'), wherein the said mobile means (2,2') for retention slide alternately in one direction and in the other, in order to approach, come into contact with and move away from the fixed means (3,3') for retention which are situated on the other side of the material in the form of a strip.

11. Machine according to any one of claims 1 to 10, **characterised in that** the means (4) for tearing are guided linearly on a plane which is perpendicular to the axis of progression (A), in slides (10,10') in which they slide alternately in one direction and in the other.

12. Machine according to claim 10 and claim 11, **characterised in that** the alternating linear motion of the mobile means (2,2') for retention of the material in the form of a strip and of the means (4) for tearing is controlled respectively by the first link mechanism (T1) and by the second link mechanism (T2).

13. Machine according to any one of claims 1 to 12, **characterised in that** the means (2,3) for retention of the strip in an immobile position are two jaws (2,2',3,3') each, on both sides of the strip of material to be divided up, which are separated by a gap in which the means (4) for tearing are situated and displaced.

14. Machine according to any one of claims 1 to 13, **characterised in that** the said means for tearing consist of at least one plate (4), one side of which comprises teeth (5) which carry out tearing perpendicularly to the axis of progression when the jaws (2,2',3,3') which are placed on both sides of the strip of material are in contact with one another via the strip, in order to keep the strip fixed.

15. Machine according to claim 14, **characterised in that** the plate (4) for tearing is of the type with a sawtooth blade.

16. Machine according to any one of claims 13 to 15, **characterised in that** the means (3,3') for fixed retention constitute at least partially the slide for guiding the means (4) for tearing, which are displaced between them and also between the mobile jaws (2,2') during the tearing step.

17. Machine according to any one of claims 14 to 16, **characterised in that** the mobile jaws (2,2') and the blade (4) for tearing are displaced in front of an opening (11) which permits passage of the material in the form of a strip to be divided up, the length of their displacement being at least equal to the width of the said opening.

18. Machine according to claim 17 for production of a wedging/padding material, **characterised in that** it comprises an output window (11) with a rectangular shape which is situated upstream from the device for separation, and through which the material in the form of a strip passes.

19. Machine according to claim 1 for production of a wedging/padding material, **characterised in that** the device for separation is secured to a panel (1) in which the said opening (11) is provided, such that the mobile means (2,2') for retention of the material in the form of a strip and the means (4) for tearing slide in front of the said opening (11) on planes which are parallel to the plane of the panel (1).

20. Method for obtaining wedging/padding material from a material in the form of a strip of the paper type, comprising at least one layer, comprising the following steps:
- longitudinal folding of the lateral edges of the strip towards the central area of the said strip;
- driving the folded assembly;
- crumpling the folded assembly; and finally
- assembling the crumpled assembly by means of successive selective compressions,
**characterised in that** the final step consists of carrying out cutting of the said material by means of a device for separation which is provided with means for tearing the section situated downstream from the said device, the device comprising means for tearing the section situated downstream from the said device for separation, these means for tearing operating perpendicularly to the said axis of progression (A) by tearing of the said padded strip, and comprising means (2,2',3,3') for keeping the material in the form of a strip firmly in an immobile position at least in the area of tearing, which means are disposed on both sides of the means (4) for tearing which are applied to the said material in the form of a strip in order to separate the section situated downstream from the said device for separation, the strip advancing according to an axis of progression (A) which is perpendicular to the said device.
